Europäisches Patentamt

European Patent Office

Office européen des brevets

(11) Numéro de publication: **0 018 276**
**A1**

(12) # DEMANDE DE BREVET EUROPEEN

(21) Numéro de dépôt: 80400486.9

(22) Date de dépôt: 14.04.80

(51) Int. Cl.³: **A 61 B 6/00**, A 61 B 6/08, A 61 B 6/02, G 03 B 41/14

(30) Priorité: 23.04.79 FR 7910211

(43) Date de publication de la demande: 29.10.80 Bulletin 80/22

(84) Etats contractants désignés: **DE GB SE**

(71) Demandeur: **COMPAGNIE GENERALE DE RADIOLOGIE, 13 square Max-Hymans, F-75741 PARIS CEDEX 15 (FR)**

(72) Inventeur: **Bens, Jean, "THOMSON-CSF" - SCPI 173, bld Haussmann, F-75360 Paris Cedex 08 (FR)**
Inventeur: **Bloch, Marcel, "THOMSON-CSF" - SCPI 173, bld Haussmann, F-75360 Paris Cedex 08 (FR)**
Inventeur: **Chekroun, René, "THOMSON-CSF" - SCPI 173, bld Haussmann, F-75360 Paris Cedex 08 (FR)**
Inventeur: **Klausz, Rémy, "THOMSON-CSF" - SCPI 173, bld Haussmann, F-75360 Paris Cedex 08 (FR)**

(74) Mandataire: **Thrierr, Francoise et al, "THOMSON-CSF" - SCPI 173, bld Haussmann, F-75360 Paris Cedex 08 (FR)**

(54) **Dispositif pour le repérage anatomique précis de coupes tomodensitométriques.**

(57) Dispositif permettant de repérer de façon précise et sans abiguïté des coupes tomodensitométriques pouvant être inclinées d'une façon quelconque par rapport à un plan origine $P_O$ prédéterminé.

Le dispositif de l'invention comporte au moins un élément de repérage (A) disposé sur la partie à examiner par un tomodensitomètre classique, cet élément ayant une ligne de base située dans le plan origine $P_O$ et ayant au moins une caractéristique variant de façon continue ou quasi-continue le long d'une direction H perpendiculaire à cette base, la variation de cette caractéristique étant mesurable sur les images tomodensitométriques des différentes coupes; cette caractéristique peut être notamment une dimension de l'élément (A): largeur perpendiculaire à H dans le cas où A est un triangle.

EP 0 018 276 A1

1

# DISPOSITIF POUR LE REPERAGE ANATOMIQUE PRECIS DE COUPES TOMODENSITOMETRIQUES.

La présente invention concerne un dispositif permettant de repérer facilement et avec grande précision des coupes tomodensitométriques réalisées sur un sujet à examiner, ce repérage étant fait par rapport à des plans anatomiques prédéterminés et classiques du sujet.

Les appareils de tomodensitométrie sont maintenant bien connus et ne seront pas décrits ici. Il suffit, pour comprendre l'objet de la présente invention de savoir que, quel que soit le type de tomodensitomètre envisagé, celui-ci permet d'obtenir des images des différentes tranches du patient examiné. Ces tranches d'examen, appelées coupes tomodensitométriques, sont généralement perpendiculaires à l'axe longitudinal (ou grand axe) du sujet à examiner. Elles peuvent toutefois être inclinées par rapport à cet axe. Ceci est notamment le cas lorsqu'il s'agit de réaliser des coupes tomodensitométriques de la tête. Dans ce cas les coupes peuvent être parallèles au plan orbito-méatal, c'est-à-dire dans un plan faiblement incliné par rapport à un plan normal à l'axe longitudinal du patient ; elles peuvent également être parallèles au plan frontal du sujet, c'est-à-dire perpendiculaires aux coupes définies précédemment.

Il peut encore dans certains cas être utile de réaliser des coupes inclinées par rapport aux différents plans que l'on vient de définir.

Il est classique d'équiper les tomodensitomètres de dispositifs de repérage qui permettent à l'opérateur de reconnaître les coupes qu'il est en train d'effectuer, aussi bien les unes par rapport aux autres, que par rapport à l'anatomie du patient examiné.

Il existe actuellement différents types de dispositifs de repérage ; deux de ces types de dispositif sont brièvement rappelés ici.

Le brevet américain 4.117.337 décrit un dispositif de repérage utilisant des centreurs lumineux qui, à partir de réglages préalables relativement complexes, permettent de définir sur le sujet à examiner un plan qui sera le plan de référence. Toutes les coupes réalisées par la suite seront repérées par rapport à ce plan de référence. Un tel système présente, outre sa complexité, différents inconvénients. D'une part il nécessite un réglage préalable qui est relativement long à réaliser. D'autre part, définissant un plan de référence qui n'est pas lié au sujet à examiner, mais au dispositif centreur optique, il nécessite une immobilité quasi absolue du patient sous peine de perdre toute son efficacité, et doit être refait à chaque nouvel examen, ce qui est un inconvénient important.

Un autre type de dispositif de repérage est décrit dans le brevet américain 4.115.691. Ce dispositif, beaucoup plus simple que le précédent, consiste essentiellement en un petit cylindre que l'on accole au sujet à examiner. Ce petit cylindre comporte différentes sections juxtaposées le long des différentes coupes à effectuer. Chaque section comporte un chiffre qui sera visualisé dans l'image tomodensitométrique et qui permet d'identifier les différentes coupes les unes par rapport aux autres. Ce dispositif, s'il est beaucoup plus simple que le précédent, présente cependant des inconvénients. Il est en effet indispensable que les coupes soient normales au cylindre contenant les chiffres de repérage. Il faut de plus que la

3

première coupe soit convenablement positionnée par rapport à ce cylindre, de manière à ne pas être à cheval sur deux petites sections adjacentes, ce qui rendrait la lecture des deux chiffres impossible. Un tel dispositif nécessite donc un réglage préalable de la première coupe par rapport à la longueur du petit cylindre. De plus il ne permet pas de réaliser des coupes selon des incidences quelconques.

L'objet de la présente invention est un dispositif permettant de repérer facilement les différentes coupes par rapport à l'anatomie du sujet à examiner, ne nécessitant pas de réglage préalable du positionnement des coupes, et permettant de repérer convenablement des coupes présentant des inclinaisons différentes par rapport à un plan de référence quelconque.

Selon l'invention, un dispositif pour le repérage anatomique précis des coupes tomodensitométriques réalisées sur un sujet par un tomodensitomètre, comporte au moins un élément allongé, disposé sur le sujet de façon reproductible, cet élément étant constitué, au moins en partie, d'un matériau visible sur les coupes tomodensitométriques, et ayant au moins une caractéristique dont la grandeur varie, le long de sa grande dimension (direction L), toujours dans le même sens et de façon continue ou quasi continu, cet élément étant visualisé sur les images tomodensitométriques des coupes à repérer par une trace sur laquelle est effectuée la mesure de la grandeur variable, de manière à obtenir le repérage des coupes le long de L.

Un avantage important du dispositif de l'invention est, outre sa grande simplicité, de pouvoir être utilisé quelle que soit la position des coupes le long de la direction L, ceci étant possible par le fait que la caractéristique qui va permettre le repérage varie de façon continue le long de L.

D'autres objets caractéristiques et résultats de l'invention ressortiront de la description suivante donnée à titre d'exemple non limitatif, et illustrée par les figures annexées qui représentent :

- la figure 1, une représentation d'un mode de réalisation d'un élément du dispositif de repérage selon l'invention ;

- la figure 2, un exemple d'utilisation de dispositif de repérage selon l'invention comportant deux éléments tels que celui de la figure 1 ;

- la figure 3, le représentation schématique d'une coupe tomodensitométrique réalisée avec un dispositif de repérage conforme à celui de la figure 2 ;

- les figures 4(a) et 4(b), une variante de réalisation de l'élément de la figure 1 ;

- les figures 5(a), 5(b) et 5(c), des vues schématiques d'une variante perfectionnée du dispositif de l'invention ;

- les figures 6(a) et 6(b), 7(a) et 7(b), des vues schématiques de deux modes d'utilisation de la variante de la figure 5 ;

- les figures 8, 9 et 10, des schémas représentant les abaques utilisables pour obtenir les données de repérage de plans de coupes quelconques par rapport à un plan origine prédéterminé ;

- la figure 11, un schéma permettant d'expliquer un mode de calcul des données de repérage de plans de coupes quelconques par rapport à un plan origine prédéterminé.

Le dispositif de repérage de coupes tomodensitométriques conforme à l'invention permet, comme il a déjà été mentionné, de repérer des coupes entre elles, et de les repérer par rapport à l'anatomie du sujet,

5

pratiquement quelle que soit l'orientation de ces coupes. Une telle possibilité est particulièrement intéressante lorsqu'il s'agit d'examiner la tête du sujet, ce qui peut nécessiter aussi bien des coupes transverses, c'est-à-dire perpendiculaires à l'axe longitudinal du sujet, que des coupes non transverses, et par exemple parallèles à cet axe ou bien inclinées par rapport à cet axe. Il faut toutefois noter que, bien que la description qui va suivre est plus particulièrement faite à l'occasion d'examens de la tête, le dispositif de repérage de l'invention est tout aussi bien utilisable lorsqu'il s'agit de réaliser des examens d'autres parties du corps que la tête. Il suffit alors de disposer ce dispositif de repérage à l'endroit où l'on veut réaliser des examens.

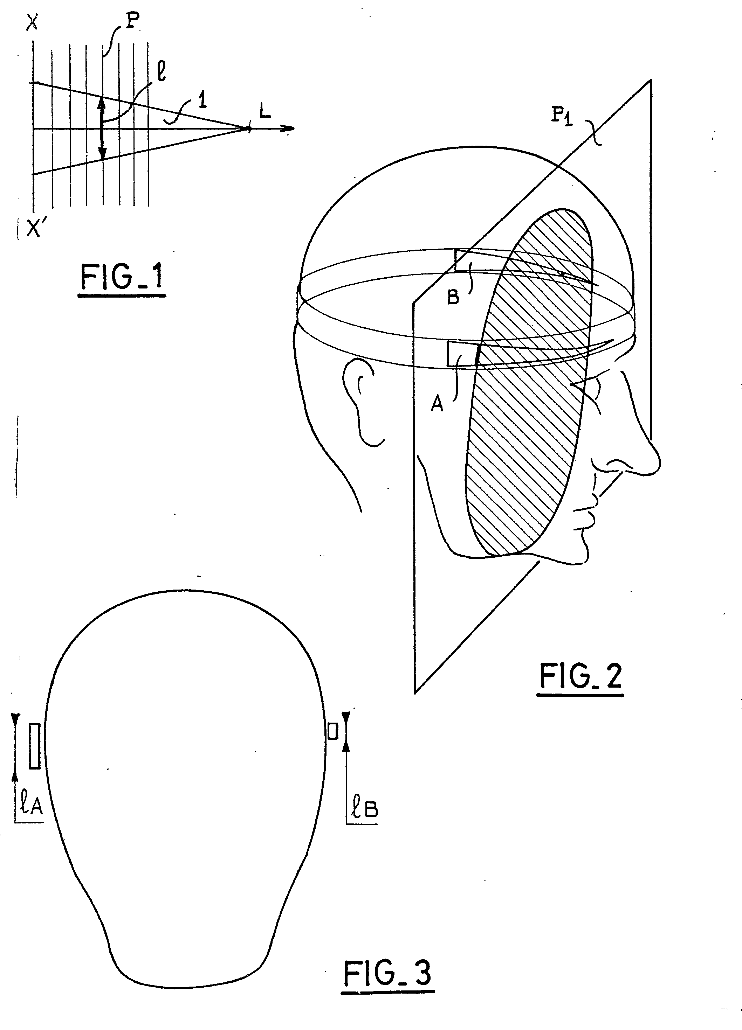

La figure 1 illustre schématiquement un élément triangulaire 1, utilisé par le dispositif de repérage de l'invention. La description de cet élément et de son mode d'utilisation, va permettre de comprendre le principe de base de l'invention, principe qui donne une grande une grande liberté dans l'utilisation du dispositif.

Un dispositif de repérage conforme à l'invention comporte au moins un élément tel que le triangle 1 disposé sur le sujet à examiner. Ce triangle 1 est ici un triangle isocèle dont le matériau constituant est choisi pour sa masse volumique comprise entre 1 et 1,2 par exemple, de façon à apparaître nettement dans l'image tomodensitométrique des coupes, sans introduire d'artéfact.

L'élément constitué par le triangle 1 a, le long de la direction L, une caractéristique dont la grandeur varie de façon continue en fonction de la position le long de L. Cette caractéristique est la largeur 1 de triangle coupé par des droites parallèles à l'axe XX'. L'axe XX' confondu ici avec la base du

6

triangle 1, constitue un axe de référence. L'axe de référence peut être toute droite parallèle à la base du triangle et distincte de cette base ; l'axe de référence XX' sera, par la suite, appelé "ligne de base" ; l'angle du triangle, formé par les deux côtés se coupant le long de la direction L sera appelé "angle au sommet".

Si l'on effectue des coupes tomodensitométriques de ce triangle parallèlement à l'axe XX', sa trace sur l'image tomodensitométrique est un petit rectangle dont l'épaisseur est l'épaisseur du triangle 1 et dont la hauteur, appelée largeur dans la suite du texte est fonction de l'emplacement de la coupe le long de la direction L.

L'invention consiste essentiellement à disposer sur le sujet, dans la région à examiner, au moins un élément tel que le triangle 1, et à mesurer sur l'image tomodensitométrique la largeur de la trace du triangle 1. La connaissance de la largeur de cette trace indiquera sans ambiguïté, et pour chaque position de la coupe le long de L la position exacte du plan d'examen par rapport à la ligne de base XX' du triangle 1. Il suffit de positionner le triangle 1 de manière connue, et de préférence reproductible, sur la partie examinée, pour avoir un repérage anatomique précis et facile à réaliser des coupes tomodensitométriques effectuées.

Le mesure de la dimension de cette trace fine ne présente aucune difficulté. En effet, la plupart des tomodensitomètres existant à l'heure actuelle permettent de réaliser une mesure absolue des distances sur les images qu'ils donnent. Si l'on obtient, pour une coupe dont le plan moyen intersecte le triangle 1 de la figure 1 le long de la trace P, une trace dont la dimension

7

est l, la correspondance biunivoque existant entre
la position de P le long de L et la grandeur de l indique sans ambiguïté la position de ce plan P. Il est
possible pour faciliter le repérage, de graduer directement le triangle l en indiquant le long de L, un
certain nombre de grandeurs l. La mesure des grandeurs
l sur les images tomodensitométriques, indiquera directement, par cette correspondance, la position du plan
moyen de la coupe réalisée.

Dans la description qui vient d'être faite, où
le dispositif de repérage comporte un seul élément tel
que le triangle l, le repérage est réalisé par rapport
à un axe, ici l'axe de référence XX'. Il est clair
que ceci peut être utilisé pour des coupes tomodensitométriques présentant une inclinaison quelconque
par rapport au grand axe du sujet, puisqu'il suffit
de positionner ce triangle l de manière que sa base
(c'est-à-dire l'axe XX'), soit parallèle aux plans
des coupes qui vont être réalisées. Il est clair également qu'il n'est pas besoin de réaliser de réglage
préalable de la première coupe par rapport au triangle
l. En effet, la caractéristique qui permet de déterminer la position du plan de coupe P le long de L,
variant de façon continue, une mesure précise peut
être obtenue quelle que soit la position de ce plan
de coupe le long de L. Il peut être noté encore une
fois que ceci n'était pas possible avec des systèmes
à indexation discrète tel que c'était le cas dans
le brevet américain précité 4.115.691.

Il faut toutefois noter qu'il est généralement
nécessaire de repérer les coupes tomodensitométriques
non pas par rapport à un axe tel que XX', mais par
rapport à un plan de référence. Le dispositif de repérage de l'invention se prête très facilement à de
tels repérages.

8

La figure 2 représente schématiquement un dispositif de repérage conforme à l'invention permettant de repérer un plan de coupe tomodensitométrique par rapport à un plan de référence prédéterminé. Dans l'expemple représenté sur cette figure, il s'agit de réaliser des coupes tomodensitométriques, approximativement verticales, de la tête d'un patient à examiner. Deux éléments en triangle A et B, tels que décrits précédemment, sont disposés de part et d'autre de la tête du patient (à droite et à gauche), leurs deux lignes de base parallèles définissant un plan de référence. Les deux éléments triangulaires A et B sont par exemple constitués d'un matériau présentant une certaine souplesse de façon à pouvoir suivre approximativement les contours extérieurs de la tête du sujet. Ils peuvent être fixés sur la tête par tous moyens appropriés tels que rubans adhésifs, serre-tête élastiques, bandeau etc ... Si les coupes densitométriques sont réalisées selon des plans parallèles au plan de référence défini par les deux lignes de base des triangles, les traces des deux triangles A et B sur les vues densitométriques sont égales. Par contre, si le plan de coupe $P_1$, tout en restant parallèle aux lignes de base des deux triangles, n'est plus parallèle au plan de référence, les traces ne seront plus égales, comme illustré sur la figure 3. Les largeurs $l_A$ et $l_B$ de ces deux traces donnent une indication précise de la position et de l'inclinaison des plans de coupe tels que $P_1$ par rapport au plan de référence. Le rapport de ces deux largeurs est caractéristique de l'inclinaison du plan de coupe $P_1$ par rapport au plan de référence. Leur grandeur respective est caractéristique de la distance du plan de coupe au plan de référence. La mesure de ces deux grandeurs indique donc de façon

9

précise et sans aucune ambiguïté la position de chaque coupe par rapport à l'anatomie du sujet.

Il est particulièrement commode, après avoir réalisé un premier ensemble de coupes tomodensitométriques ainsi repérées, d'en réaliser un second. Il n'est pas nécessaire de refaire les opérations de repérage. Il est également très commode de passer d'une opération d'examen radiologique à une opération de chirurgie. Il suffit,, soit de conserver les deux éléments triangulaires A et B en place entre les deux opérations, soit de les repositionner exactement de la même manière.

Il est à noter que plus l'ouverture de l'angle au sommet des triangles de repérage est grande, plus la précision de repérage est importante.

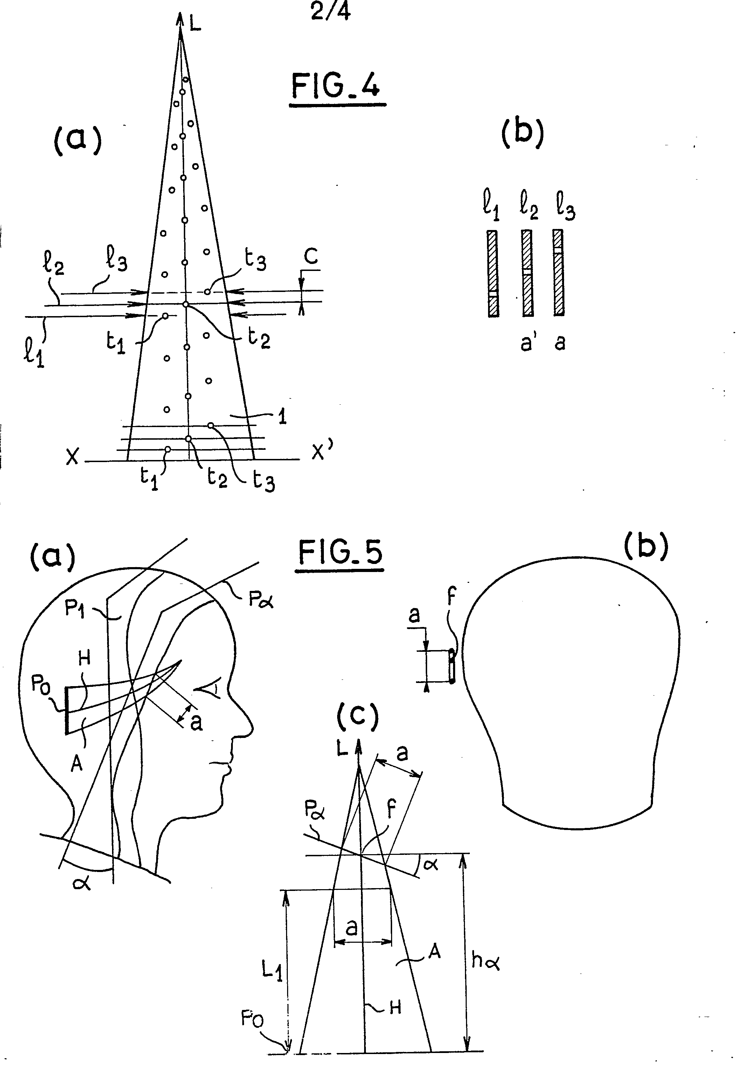

Les figures 4a et 4b indiquent schématiquement une variante de réalisation des éléments triangulaires tels que A et B permettant d'augmenter encore la précision de la mesure. Il suffit pour cela de disposer le long des différentes traces parallèles à l'axe XX' des repères matériels qui apparaîtront distinctement sur les traces du triangle dans les vues tomodensitométriques. Ces repères sont disposés comme indiqué sur la figure 4a par exemple, à savoir un repère par trace, et sont décalés, d'une trace à la suivante, d'une distance qui apparaîtra nettement sur les traces des vues tomodensitométriques (figure 4b). Dans l'exemple illustré ici ces repères consistent en des petits trous pratiqués dans le triangle ; dans ce même exemple, où les trous sont groupés par groupes de trois $(t_1, t_2, t_3)$, trois largeurs voisines $l_1$, $l_2$, $l_3$, de valeur peu différente, ce qui aurait pu conduire à les confondre sur les vues tomodensitométriques, sont bien différenciées par la position dans leur largeur de la trace des trous $t_1$, $t_2$ et $t_3$. Si c est la distance entre deux trous successifs d'un même groupe, et si d
est le diamètre de ces trous, l'erreur maximale est
alors : $\pm \frac{c}{2} - d$.

Il est à noter qu'il est possible de réaliser
une variante des éléments de repérage en utilisant,
comme caractéristique variant de façon continue le
long de la direction L, la densité du matériau constituant l'élément de repérage. Dans ce cas l'élément
peut être constitué d'une simple réglette allongée,
rectangulaire par exemple, dont la longueur est la
direction L et dont la largeur, perpendiculaire à L,
définit une "ligne de base". Dans ce cas, c'est la
variation de densité qui indique la position de la
trace du plan de coupe. Cette variante conserve le
double intérêt de ne pas nécessiter de réglage préalable puisqu'une trace peut être repérée où qu'elle
soit le long de la direction L, et celui de permettre
le repérage de plans inclinés d'angles variables par
rapport au plan défini par les lignes de base de deux
telles réglettes disposées de part et d'autre du sujet à examiner.

Il est possible, en combinant une variation de largeur 1 de l'élément de repérage, et une variation
de densité, de parvenir à une précision améliorée, en
combinant le résultat des deux mesures. Pour cela, les
éléments de repérage sont des éléments triangulaires
comme déjà décrits et le matériau qui les constitue
varie de façon continue, ou quasi continue, le long
de leur direction L. Dans ce cas les images tomodensitométriques contiennent des traces des triangles dont
la largeur est fonction de la position de la trace du
plan de coupe le long de L, et dont l'intensité sur
l'image visualisée est également fonction de cette
position. Il suffit alors de combiner les informations

11

de dimensions de ces traces (comme indiqué précédemment), avec les informations données par la mesure de la densité radiologique des traces des éléments sur les coupes tomodensitométriques reconstruites.

Différentes méthodes, classiques en elles-mêmes, sont utilisables pour obtenir un triangle présentant une telle variation continue ou quasi continue de densité le long de la direction L. Une première méthode consiste à incorporer dans le matériau constituant le triangle une poudre d'un matériau de densité différente, la proportion de cette poudre variant de façon continue de la base du triangle à sa pointe. Une autre méthode consiste à réaliser ce triangle à partir de plusieurs petits triangles élémentaires disposés côte à côte de façon imbriquée (tête-bèche), de manière que la base de l'un soit voisine de la pointe de l'autre, ces triangles étant consitués de matériaux présentant des densités différentes.

Dans les exemples illustrés aux figures 1 et 2 les triangles sont des triangles isocèles ; cette forme n'est pas, dans ces variantes, indispensable. Il peut très bien s'agir de triangles non isocèles, rectangles par exemple, ou même d'autres formes géométriques allongées que des triangles.

Dans l'exemple de la figure 2, les plans de coupe sont approximativement parallèles à l'axe longitudinal du sujet. Si l'on veut réaliser des coupes perpendiculaires à cet axe, c'est-à-dire des coupes transverses, il suffit de disposer les triangles A et B à 90° de la position indiquée sur la figure 2.

Dans toutes les variantes de réalisation de l'invention qui viennent d'être décrites, s'il est

12

possible de repérer des plans de coupes présentant une certaine inclinaison par rapport à un plan de référence, il n'est pas possible de repérer avec précision.des plans de coupes inclinés de manière quelconque. En effet, il a été dit jusqu'à présent qu'il était nécessaire que les plans de coupes restent toujours parallèles à la ligne de base des éléments de repérage tels que les triangles A et B. En effet, si les triangles n'étaient plus coupés selon des traces parallèles à leurs lignes de base, les informations obtenues seraient érronées. D'autres variantes vont maintenant être décrites qui permettent de repérer avec toute la précision voulue des indidences quelconques.

Les figures 5a, 5b et 5c permettent d'expliquer le perfectionnement apporté par ces variantes.

On voit sur la figure 5a un triangle A fixé sur la tête 2 d'un patient ; la base de ce triangle A est approximativement parallèle à l'axe longitudinal du patient. Cette base définit, avec celle d'un second triangle identique B, non visible sur la figure, mais disposé identiquement de l'autre côté du patient, un plan de référence approximativement vertical dans cet exemple. Ce plan $P_o$ sera pris comme plan origine sur le patient. Tant que l'on réalise des coupes tomodensitométriques selon des plans tels que $P_1$ parallèle au plan origine $P_o$, les triangles utilisés précédemment sont suffisants et la trace a (figure 5b) de ces triangles indique avec précision la position de $P_1$ par rapport à $P_o$. Par contre, lorsqu'il réalise des coupes tomodensitométriques qui ne sont plus parallèles à la base du triangle A, mais qui sont inclinées d'un angle $\alpha$ par rapport au plan origine $P_o$ la mesure de la trace a (figure 5b) du triangle sur l'image tomodensitométrique, ne donne plus une information convenable quant au repérage du plan $P\alpha$.

La figure 5c permet de comprendre comment une variante du dispositif de repérage de l'invention permet d'obtenir, malgré cela, un repérage convenable et précis de la distance du plan $P\alpha$ par rapport au plan origine $P_0$ et de l'inclinaison $\alpha$ de ce plan $P\alpha$.

Si l'on se contentait, réalisant une coupe selon un plan $P\alpha$, de mesurer la largeur a de la trace du triangle A, on obtiendrait, comme l'indique la figure 5a, un repérage de ce plan $P\alpha$ indiquant qu'il s'agit d'un plan de coupe coupant le triangle A selon la trace a parallèle à sa base et distante de cette base de la longueur $L_1$ (figure 5c). La mesure correcte devrait indiquer qu'il s'agit d'un plan coupant le triangle A selon la trace $P\alpha$ indiquée sur la figure 5c. Cette trace $P\alpha$ est inclinée d'un angle $\alpha$ par rapport à la base du triangle, la trace a se retrouvant alors entre les deux côtés du triangle, sur $P\alpha$. Un repérage convenable doit donc donner les deux informations : angle $\alpha$ ; hauteur $h\alpha$ par rapport à la base du triangle correspondant au plan origine $P_0$ sur le patient. Il apparaît clairement que si l'on utilisait les variantes précédemment décrites, le repérage du plan de coupe par rapport au plan origine serait d'autant plus érroné que le plan de coupe $P\alpha$ serait incliné par rapport à ce plan origine.

Les variantes permettant de repérer convenablement de tels plans $P\alpha$ consistent à utiliser un ou plusieurs éléments de repérage de forme triangulaire tel que le triangle A, susceptibles de donner, en plus de la dimension de la trace du triangle sur le plan de coupe, une information supplémentaire dont peut être déduite l'inclinaison $\alpha$ . Cette information sera déduite de repères supplémentaires situés à l'intérieur de la surface du triangle A, ces repères étant eux-mêmes visibles dans la trace du triangle sur l'image tomodensitométrique. Un repère particulièrement commode pour obtenir cette information de l'inclinaison $\alpha$ , consiste à matérialiser la hauteur H du triangle (confondue avec la direction L précédemment décrite), c'est-à-dire la bissectrice de son angle au sommet.

Cette hauteur H peut être par exemple matérialisée par un fil métallique collé le long de la hauteur H sur le triangle A. Ce fil métallique ayant une densité différente de celle de la matière constituant le triangle A, apparaîtra sur la trace a du triangle (figure 5b) sous forme d'un point f de luminosité différente du reste de la trace. Différents modes de réalisations du triangle ainsi complété seront décrits par la suite.

Lorsque le plan de coupe est parallèle au plan origine $P_O$, la trace f sera centrée au milieu de la trace a du triangle. Lorsque le plan de coupe $P\alpha$ est incliné d'un angle $\alpha$ par rapport au plan origine $P_O$, cette trace f ne sera plus au centre de la trace a. Il apparaît clairement que si l'on reporte sur le triangle de la figure 5c la trace de longueur a de manière que ses deux bords coincident avec les bords du triangle A et que la trace f coincide avec la hauteur H, une et une seule position de la trace $P\alpha$ est possible. Cette position définit l'inclinaison $\alpha$ et la hauteur $h\alpha$ de la trace du plan $P\alpha$ par rapport à la base du triangle $P_O$. Ces deux informations $\alpha$ et $h\alpha$ peuvent être obtenues comme il sera décrit plus

15

loin, soit par un calcul, soit par un système d'abaques.

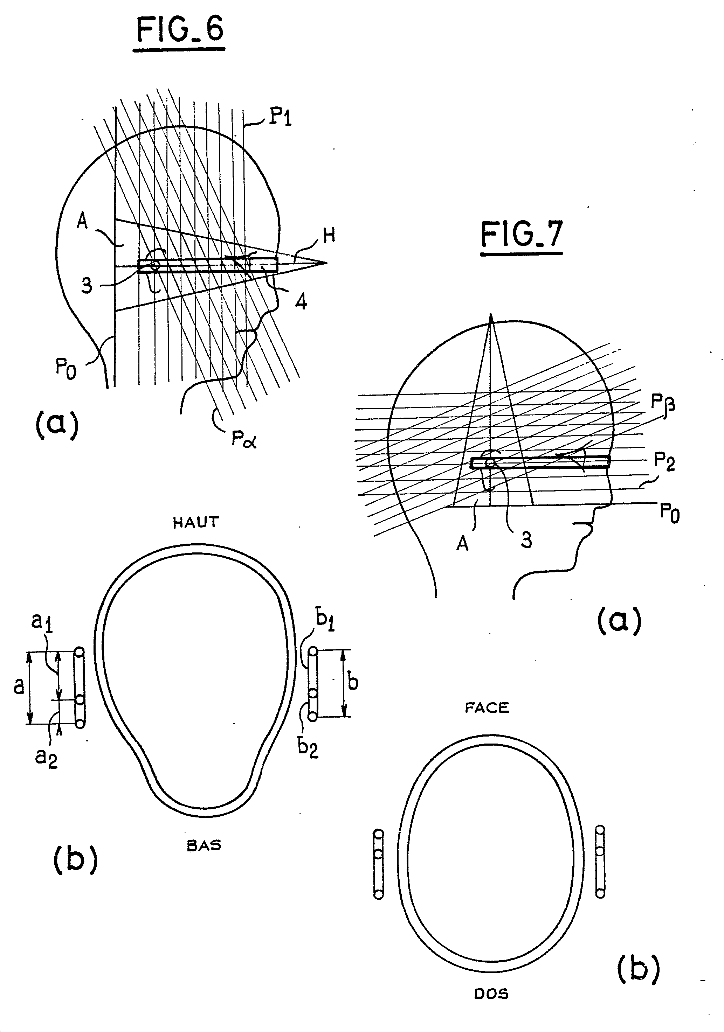

Un mode de réalisation particulier du dispositif de repérage ainsi perfectionné va maintenant être décrit à l'aide des figures 6 et 7.

Dans l'exemple de la figure 6, où il s'agit de réaliser des coupes tomodensitométriques, soit approximativement verticales, soit inclinées par rapport à la verticale, deux triangles A et B seront dis-posés de part et d'autre de la tête du patient à examiner, un seul A de ces triangles étant visible sur la figure. Ces deux triangles sont par exemple constitués de deux triangles isocèles de dimensions connues, en plexiglas, sur lesquels on a collé des fils métalliques d'un diamètre suffisamment faible pour ne pas créer d'artéfact sur les coupes tomodensitométriques et suffisamment élevé pour que les fils soient parfaitement distingués du plexiglas sur l'image de la coupe densitométrique. Ces fils de métal qui peuvent, dans un exemple, être constitués de fil de cuivre d'un diamètre de 0,4 mm, sont au nombre de trois par triangle et sont collés le long des deux côtés égaux des triangles isocèles et sur les bissectrices des angles au sommet de ces triangles. Les deux fils latéraux matérialisent les bords du triangle A ; leur distance sur la coupe tomodensitométrique indique la grandeur a de la trace du triangle. Le fil central correspond à la hauteur H du triangle ; sa trace f donne l'information supplémentaire nécessaire pour obtenir le repérage correct tant en distance qu'en angulation. Il est à noter que les fils latéraux peuvent être fixés à une certaine distance des bords du triangle support en plexiglas, et même que le support (non visible sur

16

les images reconstruites) peut être de forme quel-conque ; ce sont en effet les fils latéraux qui définissent les bords de l'élément de repérage. Il est encore possible, si les fils sont suffisamment rigides, de ne pas avoir de support ; dans ce cas les fils sont par exemple soudés entre eux et maintenus, à l'arrière (base du triangle) par une simple tige.

Les deux triangles de plexiglas A et B sont fixés sur des embouts 3 qui s'engagent dans les trous auditifs externes du sujet à examiner, ces deux embouts étant eux-mêmes solidaires d'une pièce 4, en plexiglas par exemple. La pièce 4, en arceau, prend appui sur le nasion du sujet et est immobilisée par les deux embouts 3 engagés dans les oreilles du sujet. Les deux triangles A et B sont positionnés autour des embouts 3 de manière que la hauteur H des triangles A et B (matérialisée par le fil central) soit confondue avec le plan orbito-méatal du sujet. La base des triangles A et B est alors perpendiculaire à ce plan orbito-méatal. Les deux bases ou lignes de base qui peuvent, comme déjà dit, être distinctes des bases des triangles, définissent un plan origine $P_O$ sur le patient. Sur des coupes tomodensitométriques parallèles au plan origine $P_O$ (coupes dont les traces sont repérées par les droites parallèles $P_1$ sur la figure 6$_a$), les images tomodensitométriques reconstituées feront apparaître de part et d'autre du crâne du sujet trois points correspondant aux bords du triangle et à sa hauteur, le point central étant équidistant des points externes. La distance entre les points externes de chaque trace indique la distance du plan de coupe $P_1$ par rapport au plan origine $P_O$. Si ces distances ne sont pas égales sur les deux traces situées de part et d'autre de la coupe du crâne, c'est que le plan de coupe est incliné droite-gauche comme il a été expliqué à l'occasion des figures 2 et 3.

Dès qu'il s'agit de plans de coupe $P\alpha$ inclinés d'un angle $\alpha$ par rapport au plan origine $P_O$ (les traces de tels plans étant repérés par les lignes parallèles $P\alpha$ sur la figure 6a) l'image tomodensitométrique comporte de part et d'autre du crâne du sujet deux traces comportant chacune trois points, le point central n'étant plus équidistant des deux points externes (figure 6b). Ici encore, si les plans de coupe $P\alpha$ sont, de plus, inclinés droite-gauche, les deux traces ne sont pas égales.

Les figures 7a et b illustrent l'utilisation du même dispositif que celui qui vient d'être décrit comportant deux triangles en plexiglas munis de trois fils, monté sur un arceau fixé dans les trous auditifs du sujet et sur son nasion ; mais ici les deux triangles A et B sont orientés à 90° de ce qu'ils étaient dans la figure 6. Ceci permet de repérer avec précision des coupes tomodensitométriques parallèles au plan de référence anatomique du sujet (traces parallèles $P_2$ sur la figure 7a). Ce dispositif permet également de repérer des plans de coupe inclinés d'un angle $\beta$ par rapport au plan origine sur le sujet, qui est cette fois un plan $P_O$ parallèle au plan orbito-méatal précédemment défini. Ce plan origine $P_O$ peut d'ailleurs être confondu avec le plan orbito-méatal.

Dans l'exemple qui vient d'être décrit, les deux triangles sont des triangles en plexiglas munis de trois fils métalliques. Il est clair que toute variante permettant de visualiser sur les images tomodensitométriques les bords et la hauteur du triangle est conforme à l'invention. Il est par exemple possible d'utiliser des triangles en un matériau directement visible sur les images tomodensitométriques, et de réaliser le long de leur hauteur H un évidement qui apparaîtra lui-même sur les images tomodensitométriques. Il est encore possible d'utiliser un triangle en un matériau visible sur les images tomodensitométriques, et de disposer le long de la hauteur H un fil d'une densité différente, sans avoir de fils sur les bords du triangle.

Il est à noter que le dispositif de fixation sur le sujet des deux triangles qui vient d'être décrit (arceau 4 et embouts 3) est utilisable aussi bien pour les variantes précédemment décrites : triangles sans fils et rectangles à densité variable.

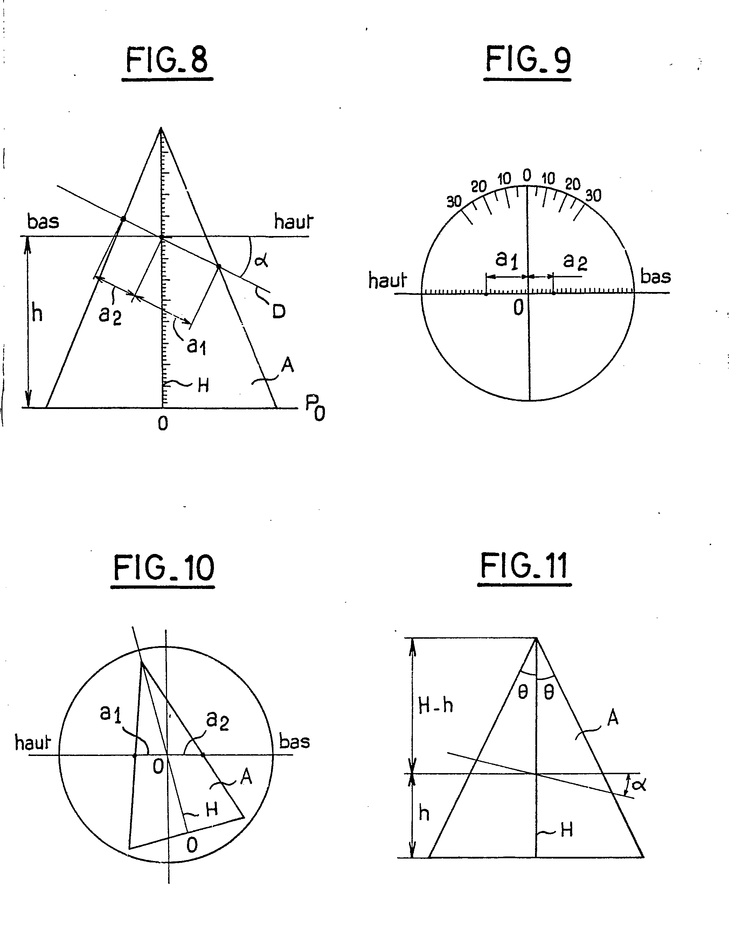

On va maintenant décrire à l'aide de la figure 8 une méthode utilisant des abaques pour obtenir rapidement, à partir des traces du dispositif de repérage de l'invention visibles sur les images tomodensitométriques, les informations de distance et d'angulation des plans de coupe par rapport au plan d'origine.

Après qu'une coupe tomodensitométrique ait été réalisée, calculée, et visualisée par les moyens classiques des tomodensitomètres, il suffit de traiter l'une après l'autre les traces a et b de la figure 6 par exemple de la manière suivante, pour obtenir le repérage précis d'un plan de coupe P par rapport au plan origine $P_O$ de la figure 6a.

Les figures 8, 9 et 10 illustrent schématiquement comment il est possible de repérer un plan de coupe quelconque par rapport au plan origine à l'aide d'abaques d'utilisation relativement simple.

Le principe de base de ces abaques consiste à superposer à un triangle A (figure 8) reproduisant exactement le triangle A disposé sur le patient, une droite D sur laquelle sont reportées les deux longueurs

19

$a_1$ et $a_2$ bout à bout. Il suffit de faire coïncider les trois points représentant les extrémités des longueurs $a_1$ et $a_2$ avec les deux côtés et la hauteur du triangle isocèle A pour obtenir en h la hauteur de la coupe et en $\alpha$ l'incidence de cette coupe. Ce report de la droite D et des deux grandeurs $a_1$ et $a_2$ sur le triangle peut se faire facilement en utilisant un rapporteur d'angle circulaire illustré sur la figure 9. Le centre du rapporteur de la figure 9 correspond au zéro. On reporte de part et d'autre de ce zéro, sur le diamètre millimétré, les longueurs $a_1$ et $a_2$ en repérant lesquelles correspondent respectivement au haut et au bas de la coupe tomodensitométrique. On vient superposer sur ce rapporteur (comme indiqué figure 10) le triangle de la figure 8, qui est réalisé en matériau transparent tel que du plexiglas, et dont la hauteur H est graduée en millimètres, la base de la hauteur H correspondant au point zéro du plan origine $P_0$. On réalise cette superposition du triangle sur le rapporteur en obligeant la hauteur H à passer par le centre O du rapporteur ; on fait coïncider les deux côtés du triangle avec les extrémités des longueurs $a_1$ et $a_2$ reportées sur le diamètre du rapporteur. Cette superposition étant réalisée, on lit la hauteur de coupe sur la hauteur H graduée du triangle, à l'intersection de cette hauteur et du diamètre gradué du rapporteur ; on lit l'angulation de la coupe sur le rapporteur, en face du sommet du triangle isocèle A.

Si les deux traces a et b de la figure 6b sont identiques, c'est que la trace du plan de coupe est la même sur les deux triangles et que ce plan conserve la symétrie bilatérale de la tête du sujet examiné. Il suffit donc de faire une fois cette opération d'abaque pour obtenir le repérage précis du plan de coupe. Si

20

par contre les deux traces sont différentes il faut réaliser deux fois cette opération sur les deux informations a et b pour obtenir la position du plan de coupe, dont la trace est différente sur les deux triangles A et B.

L'opération est la même lorsqu'il s'agit de repérer les plans $P\beta$ de la figure 7. La seule différence est que l'inclinaison n'est plus haut-bas, mais face-dos.

Cette méthode de détermination des paramètres de repérage du plan de coupe par des abaques n'est pas extrêmement précise, puisqu'il faut superposer trois points sur trois droites par tâtonnement. Il est possible d'obtenir une précision bien supérieure en opérant par calcul à partir des mesures $a_1$ et $a_2$ (et éventuellement $b_1$ et $b_2$ lorsqu'elles sont différentes). Si $\theta$ est le demi-angle au sommet du triangle isocèle (figure 11), la hauteur de coupe h par rapport au plan origine (base du triangle) et l'angulation de coupe, s'expriment mathématiquement en fonction de $a_1$, $a_2$ et des éléments du triangle A par les formules suivantes :

$$(H - h) = \frac{2\ a1\ a2}{\sqrt{(a_1-a_2)^2 + tg^2\theta\ (a_1+ a_2)^2}}$$

$$tg\ (\frac{\pi}{2} - \alpha) = tg\theta\ \frac{a1 + a2}{a1 - a2}$$

Il est alors possible, à l'aide d'une petite calculatrice, de programmer le calcul de h et $\alpha$ en entrant dans la calculatrice les valeurs $a_1$, $a_2$ et $\theta$ ; on peut également utiliser un ordinateur et par exemple un ordinateur faisant partie du système tomodensitométrique, ou encore prévoir une fonction préprogrammée sur la console d'analyse et de visualisation des images.

Il a déjà été dit que si l'on désirait obtenir complètement les informations définissant un plan de coupe quelconque, il était nécessaire de disposer des triangles de part et d'autre de la partie à examiner, et notamment de la tête. Il est à noter que ces deux triangles peuvent être disposés un peu différemment ; il peut par exemple être utilisé un triangle latéral comme illustré sur les figures 6 et 7 et un triangle frontal.

Il est encore possible, pour augmenter la précision, notamment dans les coupes situées vers les pointes des triangles où les traces sont les plus petites et donc les mesures plus difficiles, de combiner côte à côte deux triangles ; un premier triangle ayant sa pointe juxtaposée à la base du second de façon à constituer un parallèlogramme avec sa diagonale matérialisée.

Si l'on désire obtenir une donnée supplémentaire pour repérer complètement la position des plans de coupe dans l'espace, il suffit d'ajouter, aux deux éléments de repérage de la coupe par rapport à un plan origine $P_0$, un troisième élément de repérage dont la ligne de base n'est pas parallèle à celles des deux autres.

22

## REVENDICATIONS

1. Dispositif pour le repérage anatomique précis des coupes tomodensitométriques réalisées sur un sujet par un tomodensitomètre, caractérisé en ce qu'il comporte au moins un élément allongé disposé sur le sujet de façon reproductible, cet élément étant constitué, au moins en partie, d'un matériau visible sur les coupes tomodensitométriques, et ayant au moins une caractéristique dont la grandeur varie, le long de sa grande dimension (direction L), toujours dans le même sens et de façon continue ou quasi-continue, cet élément étant visualisé, sur les images tomodensitométriques des coupes à repérer, par une trace sur laquelle est effectuée la mesure de la grandeur variable, de manière à obtenir le repérage des coupes le long de L.

2. Dispositif de repérage selon la revendication 1, caractérisé en ce que l'élément allongé a une dimension, mesurée perpendiculairement à la direction L, qui varie, le long de L, de façon continue d'une extrémité à l'autre de l'élément, de manière que la trace de cet élément sur les différentes coupes tomodensitométriques espacées le long de la direction L, ait une largeur (1, a, b) directement fonction de la position de la coupe le long de L, une correspondance biunivoque existant entre chaque telle position et chaque largeur de trace.

3. Dispositif de repérage selon la revendication 2, caractérisé en ce que l'élément allongé est délimité par deux segments de droite ayant leur origine commune sur la direction L et formant entre eux un angle appelé angle au sommet de l'élément (1), et en ce que les opérations de repérage des plans de coupe se font par rapport à une ligne de base servant de référence, qui se trouve dans le plan origine (Po) par rapport

23

auquel sont effectués les repérages des différents
plans de coupe, en mesurant la largeur de la trace de
l'élément sur les images tomodensitométriques.

4. Dispositif de repérage selon la revendication
3, caractérisé en ce que la ligne de base servant de
référence est perpendiculaire à la bissectrice de
l'angle au sommet de l'élément allongé.

5. Dispositif de repérage selon l'une quelconque
des revendications 3 ou 4, caractérisé en ce que l'élément allongé est constitué d'un triangle formé d'un
matériau de densité constante et telle que sa trace
est visible sur les images tomodensitométriques, les
différentes coupes tomodensitométriques étant toujours
parallèles à la ligne de base de ce triangle, et la
largeur de la trace qui permet d'effectuer le repérage
étant toujours mesurée parallèlement à cette ligne
de base et caractérisant, de façon biunivoque, la
distance du plan de coupe par rapport à cette ligne de
base contenue dans le plan origine Po.

6. Dispositif de repérage selon la revendication
1, caractérisé en ce que l'élément allongé est constitué
de matériaux tels que sa densité varie de façon continue le long de la direction L, et soit pratiquement
constante dans sa largeur, ou "ligne de base", le
repérage de la position des différentes coupes tomodensitométriques le long de L se faisant par mesure de
la densité radiologique de la trace de l'élément dans
les coupes tomodensitométriques reconstruites.

7. Dispositif de repérage selon la revendication
6, et l'une quelconque de revendications 3 à 4, caractérisé en ce que le repérage des coupes tomodensitométriques le long de la direction L se fait par combinaison
de la mesure de densité et de la mesure de largeur des
traces de l'élément de repérage sur les images tomoden-

24

sitométriques, de manière à obtenir une précision améliorée du repérage des différents plans de coupe tomodensitométrique parallèles à la base de l'élément.

8. Dispositif de repérage selon l'une quelconque des revendications 3 à 7, caractérisé en ce que l'élément allongé est percé de groupes de trous ($t_1$, $t_2$, $t_3$ : Fig. 4a) répartis le long de la direction L, les trous de chaque groupe étant décalés le long de la largeur de l'élément de manière à apparaître en des positions différenciées dans la largeur ($l_1$ $l_2$, $l_3$, : Fig. 4b) des traces visualisées et à améliorer la précision du repérage.

9. Dispositif de repérage selon l'une quelconque des revendications 3 à 8, caractérisé en ce qu'il comporte deux éléments de repérage disposés sur la partie du sujet à examiner, leurs lignes de base étant parallèles et définissant le plan d'origine $P_O$ par rapport auquel sont effectués les repérages des différents plans de coupe, la combinaison des informations tirées de ces deux éléments permettant d'obtenir le repérage précis de plans de coupe pouvant être inclinés d'un angle variable par rapport au plan d'origine $P_O$, mais toujours parallèles aux lignes de base des deux éléments du dispositif de repérage.

10. Dispositif de repérage selon la revendication 4, caractérisé en ce que l'élément de repérage dont la ligne de base est située dans le plan origine Po par rapport auquel sont effectués les repérages des différents plans de coupe, comporte des repères supplémentaires visibles sur sa trace dans les images tomodensitométriques, ces repères supplémentaires permettant de mesurer, en plus de la distance du plan de coupe à la ligne de base de l'élément, donnée par la largeur (a) de sa trace sur l'image, l'inclinaison ($\alpha$ ou $\beta$) de ce

plan de coupe (P$\alpha$ ou P$\beta$) avec cette ligne de base, de manière à permettre le repérage précis de plans de coupe inclinés par rapport à la ligne de base de l'élément.

11. Dispositif de repérage selon la revendication 10, caractérisé en ce que les repères supplémentaires consistent en au moins une zone fine, présentant une densité différente de celle du matériau constituant l'élément et située le long de la bissectrice de l'angle au sommet de l'élément.

12. Dispositif de repérage selon la revendication 11, caractérisé en ce que la zone fine située le long de la bissectrice de l'angle au sommet consiste en un fil fin appliqué le long de cette bissectrice.

13. Dispositif de repérage selon la revendication 10, caractérisé en ce que l'élément de repérage comporte deux fils fins formant entre eux l'angle au sommet de l'élément et un troisième fil fin le long de la bissectrice de cet angle au sommet.

14. Dispositif de repérage selon l'une quelconque des revendications 10 à 13, caractérisé en ce que la distance des différents plans de coupe (h) par rapport au plan origine Po et l'angle formé par ces plans de coupe avec le même plan origine Po sont obtenus par des moyens de calcul à partir des grandeurs mesurées sur les images tomodensitométriques et des paramètres géométriques de l'élément de repérage.

15. Dispositif de repérage selon l'une quelconque des revendications 10 à 13, caractérisé en ce qu'il lui est associé un abaque comportant un triangle identique au triangle formé par l'élément de repérage, dont la hauteur H est millimétrée, et qui est utilisé pour la détermination des valeurs h et $\alpha$ qui sont respectivement la distance du plan de coupe par rapport au plan origine Po et son inclinaison par rapport à ce même plan.

16. Dispositif de repérage selon la revendication 15, caractérisé en ce que l'abaque comporte en outre un rapporteur d'angle circulaire (Fig. 9) dont un diamètre est millimétré, la détermination des valeurs h et $\alpha$ étant obtenue par report sur le diamètre millimétré des valeurs $a_1$ et $a_2$ lues sur la trace a de l'élément de repérage sur l'image tomodensitométrique, de part et d'autre du centre de ce diamètre, et par superposition du triangle et du rapporteur constituant l'abaque, la hauteur H du triangle passant par le centre du rapporteur et ses côtés par les extrémités des grandeurs $a_1$ et $a_2$ reportées sur le diamètre de ce rapporteur ; la distance du plan de coupe par rapport au plan origine est lue sur la hauteur graduée H ; l'angulation de ce plan de coupe par rapport au plan origine est lue sur le rapporteur en face de la pointe du triangle.

17. Dispositif de repérage selon l'une quelconque des revendications 10 à 16, caractérisé en ce qu'il comporte deux éléments de repérage (A et B) disposés sur la partie du sujet à examiner, leurs lignes de base étant parallèles et définissant le plan d'origine $P_o$ par rapport auquel sont effectués les repérages des différents plans de coupe, la combinaison des informations (distance et angulation) tirées de ces deux éléments de repérage permettant d'obtenir le repérage précis de plans de coupe pouvant être inclinés de façon quelconque par rapport au plan d'origine $P_o$ et par rapport aux lignes de base des deux éléments de repérage.

18. Dispositif de repérage selon l'une quelconque des revendications 9 ou 17, caractérisé en ce que les deux éléments de repérage (A et B) sont fixés sur un arceau (4) disposé autour de la partie du sujet à examiner.

19. Dispositif de repérage selon la revendication 18, caractérisé en ce que les deux éléments de repérage sont fixés symétriquement sur les deux extrémités de l'arceau (4).

20. Dispositif de repérage selon la revendication 19, caractérisé en ce que la partie du sujet à examiner étant son crâne, les deux éléments de repérage sont fixés sur l'arceau (4) par deux embouts (3) destinés à être introduits dans les conduits auditifs du sujet tandis que la partie centrale de l'arceau (4) est posée sur le nasion du sujet, l'arceau (4) se trouvant dans le plan orbito-méatal.

21. Dispositif de repérage selon l'une quelconque des revendications 9 ou 17, caractérisé en ce qu'il comporte au moins un élément de repérage supplémentaire dont la ligne de base n'est pas parallèle à celle des deux autres éléments, de manière à obtenir une donnée supplémentaire qui permet de repérer complètement la position des différents plans de coupe dans l'espace.

0018276

X

P

ℓ

1

L

X'

FIG_1

P₁

B

A

FIG_2

ℓA

ℓB

FIG_3

2/4

FIG.4

(a)

(b)

FIG.5

(a)

(b)

(c)

3/4

0018276

FIG_6

(a)

(b)

HAUT

BAS

FIG_7

(a)

(b)

FACE

DOS

## FIG.8

## FIG.9

## FIG.10

## FIG.11

Office européen
des brevets

**RAPPORT DE RECHERCHE EUROPEENNE**

0018276
Numéro de la demande

EP 80 40 0486

| Catégorie | Citation du document avec indication, en cas de besoin, des parties pertinentes | Revendication concernée |
|---|---|---|
| | FR - A - 2 230 327 (E. EDARSKY) <br> * Page 1, lignes 1-4; page 2, lignes 14-35 et la figure unique * | 1-4,7, 11 |
| | -- | |
| | US - A - 4 088 893 (C.H. SCHROEDER) <br> * Abrégé; colonne 2, ligne 45 - colonne 4, ligne 13 et figures 1-3 * | 1,9, 18,20 |
| | -- | |
| | FR - A - 2 282 253 (SIEMENS A.G.) <br> * Page 5, ligne 31 - page 6, ligne 8 et figure 9 * | 1,11, 14 |
| | -- | |
| | FR - A - 1 477 825 (ALDERSON RE-SEARCH LABORATORIES, INC.) <br> * Page 2, colonne de gauche, lignes 24-28 et colonne de droite, lignes 45-55; page 3, colonne de gauche, ligne 37 - page 4, colonne de gauche, ligne 54 et figures 1-4 * | 1,8-10, 14,17 |
| | -- | |
| | US - A - 3 714 428 (V.T. GASAWAY) <br> * Abrégé, colonne 2, lignes 3-57, colonne 3, ligne 51 - colonne 4, ligne 34 et figures 2-4 * | 1 |
| | -- | |
| | US - A - 3 577 160 (J.E. WHITE) <br> * Abrégé, colonne 3, lignes 3-30 et 58-75; colonne 4, ligne  -/- | 1,18, 19 |

**DOCUMENTS CONSIDERES COMME PERTINENTS**

**CLASSEMENT DE LA DEMANDE (Int. Cl. 3)**

A 61 B 6/00
6/08
6/02
G 03 B 41/14

**DOMAINES TECHNIQUES RECHERCHES (Int. Cl. 3)**

A 61 B 6/00
6/02
6/08
6/14
G 03 B 41/16

**CATEGORIE DES DOCUMENTS CITES**

X: particulièrement pertinent
A: arrière-plan technologique
O: divulgation non-écrite
P: document intercalaire
T: théorie ou principe à la base de l'invention
E: demande faisant interférence
D: document cité dans la demande
L: document cité pour d'autres raisons
&: membre de la même famille, document correspondant

Le présent rapport de recherche a été établi pour toutes les revendications

| Lieu de la recherche | Date d'achèvement de la recherche | Examinateur |
|---|---|---|
| La Haye | 28-07-1980 | RIEB |

OEB Form 1503.1   06.78

Office européen
des brevets

**RAPPORT DE RECHERCHE EUROPEENNE**

00182?6

Numéro de la demande

EP 80 40 0486

-2-

| | DOCUMENTS CONSIDERES COMME PERTINENTS | | CLASSEMENT DE LA DEMANDE (Int. Cl. 3) |
|---|---|---|---|
| atégorie | Citation du document avec indication, en cas de besoin, des parties pertinentes | Revendica-tion concernée | |
| | 40 - colonne 5, ligne 39 et figures 8-16 * | | |
| D | US - A - 4 115 691 (W.H. OLDENDORF/ EMI LTD) <br><br> * Abrégé; colonne 3, ligne 61 - colonne 5, ligne 7 et figures 1-3 * | 1 | |
| | FR - A - 2 345 983 (SIEMENS A.G.) <br><br> * Page 3, ligne 22 - page 4, ligne 18 et figure 1 * | 1 | DOMAINES TECHNIQUES RECHERCHES (Int. Cl. 3) |
| | FR - A - 2 362 448 (SYNTEX USA INC.) <br><br> * Page 5, lignes 18-37; page 8, ligne 24 - page 9, ligne 14; page 10, lignes 9-39 et fi-gures 1-5 * | 6 | |
| | GB - A - 1 458 196 (R.B.W. LOWNDES) <br><br> * En entier * | 1 | |
| | FR - A - 1 535 519 (G.P. RABEY) <br><br> * Page 1, colonne de gauche, ligne 1 - colonne de droite, ligne 11; page 4, colonne de droite, ligne 3 - page 5, colonne de droite, ligne 53; page 7, colonne de droite, ligne 30 - page 8, colonne de gauche, ligne 37; page 9, co-lonne de gauche, ligne 9 - co-lonne de droite, ligne 24 et | 1,18-20 | |

./..

Office européen
des brevets

**RAPPORT DE RECHERCHE EUROPEENNE**

0018276

Numéro de la demande

EP 80 40 0486

-3-

| Catégorie | Citation du document avec indication, en cas de besoin, des parties pertinentes | Revendication concernée |
|---|---|---|
| | figures 1-5,19,20,28 * | |
| | -- | |
| D | <u>US - A - 4 117 337</u> (P.F. STAATS/ GENERAL ELECTRIC COMPANY)<br><br>* Abrégé, colonne 3, lignes 16-35; colonne 4, ligne 53 - colonne 5, ligne 16; colonne 6, ligne 44 - colonne 7, ligne 35 et figures 1-4 * | 1 |

**DOCUMENTS CONSIDERES COMME PERTINENTS**

CLASSEMENT DE LA DEMANDE (Int. Cl. ³)

DOMAINES TECHNIQUES RECHERCHES (Int. Cl. ³)